Europäisches Patentamt

European Patent Office

Office européen des brevets

(11) Veröffentlichungsnummer : **0 057 957 B1**

(12) # EUROPÄISCHE PATENTSCHRIFT

(45) Veröffentlichungstag der Patentschrift :
21.11.85

(21) Anmeldenummer : 82200102.0

(22) Anmeldetag : 28.01.82

(51) Int. Cl.⁴ : **A 61 B 6/06, G 01 N 23/04,
G 21 K 1/02, G 01 T 1/29**

(54) Vorrichtung zur nichtmedizinischen Untersuchung eines Körpers.

(30) Priorität : 06.02.81 DE 3104052

(43) Veröffentlichungstag der Anmeldung :
18.08.82 Patentblatt 82/33

(45) Bekanntmachung des Hinweises auf die Patenterteilung : 21.11.85 Patentblatt 85/47

(84) Benannte Vertragsstaaten :
CH DE FR GB LI SE

(56) Entgegenhaltungen :
EP-A- 0 001 523
EP-A- 0 003 693
EP-A- 0 037 151
DE-A- 2 512 103
FR-A- 2 326 711
US-A- 4 188 541
Patent Abstracts of Japan Band 3, Nr. 77, 30. Juni
1979 Seite 141E120
THE SOVIET JOURNAL OF NONDESTRUCTIVE TES-
TING, Band 9, Nr. 5, Juli 1974, New York S.
POPOV "Spatial Radioactive Labelling in Materials
Research" Seiten 616 bis 618
ELECTRO MEDICA, Band 49, Nr. 1 Januar 1981,
Erlangen R. MOORE et al. "Schlitzaufnahmetechnik
mit mitgeführtem Strahler" Seiten 34 bis 41

(73) Patentinhaber : Philips Patentverwaltung GmbH
Billstrasse 80
D-2000 Hamburg 28 (DE)
DE
N.V. Philips' Gloeilampenfabrieken
Groenewoudseweg 1
NL-5621 BA Eindhoven (NL)
CH FR GB LI SE

(72) Erfinder : Strecker, Helmut
Hidrehmen 37
D-2000 Hamburg 55 (DE)

(74) Vertreter : Hartmann, Heinrich, Dipl.-Ing. et al
Philips Patentverwaltung GmbH Billstrasse 80 Postfach 10 51 49
D-2000 Hamburg 28 (DE)

Jouve, 18, rue St-Denis, 75001 Paris, France

**Beschreibung**

Die Erfindung betrifft eine Vorrichtung zur nichtmedizinischen Untersuchung eines Körpers nach dem Oberbegriff des Anspruches 1.

Aus der EP-OS 0 001 523 ist bereits ein Computertomograph bekannt, bei dem ein zu untersuchender Körper mit einem fächerförmigen Strahlenbündel durchstrahlt wird. Um eine Durchstrahlung des Körpers bei erhöhtem räumlichen Auflösungsvermögen vornehmen zu können, befindet sich im Strahlengang eine Blendenanordnung, deren Blendenöffnungen entsprechend dem gewünschten Auflösungsvermögen in ihrer Größe verändert werden können. Der Körper bzw. Untersuchungsbereich wird dann bei einer Stellung der Strahlenquelle bzw. Detektoranordnung relativ zum Körper entlang von Strahlenwegen durchstrahlt, die nicht direkt aneinanderliegen, sondern zwischen denen sich jeweils Körperbereiche befinden, die bei dieser Stellung nicht durchstrahlt werden. Um die Körperabsorption auch in diesen Bereichen messen zu können, muß die Strahlenquellen-Detektoranordnung relativ zum Körper gedreht werden. Die bekannte Anordnung gestattet es somit bei gewünschtem hohem örtlichem Auflösungsvermögen nicht, den Untersuchungsbereich des Körpers lückenlos zu erfassen, wenn dieser von dem fächerförmigen Strahlenbündel in nur einer Richtung durchstrahlt wird.

Weiterhin ist aus den Patent Abstracts of Japan, Vol. 3, Nr. 77, 30. Juni 1979, S. 141 E 120, Absatz 2 ein Computertomograph bekannt, bei dem eine Anzahl von Detektoren nebeneinander angeordnet ist, die ein fächerförmiges Strahlenbündel erfassen. Zwischen der Strahlenquelle und der Detektoranordnung ist eine Blendenanordnung vorgesehen, durch die jedem Strahlungsdetektor eine Blendenöffnung zugeordnet wird, die ein Abtaststrahlenbündel mit einem Öffnungswinkel hindurchläßt, der senkrecht zum fächerförmigen Strahlenbündel kleiner ist als der durch den zugeordneten Strahlungsdetektor und die Röntgenstrahlen definierte Meßwinkel. Dabei wird während der Messung eine Translationsbewegung zwischen dem zu untersuchenden Körper und der Blendenanordnung ausgeführt.

Mit der bekannten Anordnung ist es jedoch nur möglich, jeweils ein eindimensionales Projektionsbild zu erzeugen.

Aufgabe der vorliegenden Erfindung ist es, eine Anordnung der eingangs genannten Art so auszugestalten, daß ein zweidimensionales Projektionsschattenbild erzeugt werden kann.

Diese Aufgabe wird erfindungsgemäß durch die im Kennzeichen des Hauptanspruches angegebenen Maßnahmen gelöst.

Bei der Erfindung erfolgt also zwischen dem Körper und der Blendenanordnung eine Relativverschiebung in zwei zueinander senkrechten Richtungen z. B. mäanderförmig. Dadurch wird erreicht, daß bei einer Bestrahlung des Körpers mit einem kegelförmigen Primärstrahlenbündel der zu untersuchende Körperbereich praktisch lückenlos mittels der von den einzelnen Blendenöffnungen ausgeblendeten Abtaststrahlenbündel abgetastet wird, wobei die Absorption entlang der durch die Blendenöffnungen verlaufenden Strahlenwege gemessen werden kann, so daß ein zweidimensionales Projektionsbild des Körpers erzeugt wird.

Für den Fall, daß der Körper mit hohem örtlichen Auflösungsvermögen abgetastet werden soll, können die Blendenöffnungen so weit verkleinert werden, daß die Öffnungswinkel der Abtaststrahlenbündel erheblich kleiner sind als der Meßwinkel.

Die bei jeder Stellung der Blendenanordnung relativ zum Körper von jedem Detektor gemessenen Absorptionswerte werden in einem elektronischen Speicher gespeichert. Danach werden die Absorptionswerte unter Berücksichtigung der Stellung der Blendenanordnung relativ zum Körper mittels eines Rechners einer Bildwiedergabeeinheit zugeführt, auf der das Projektionsbild des Körpers dargestellt wird.

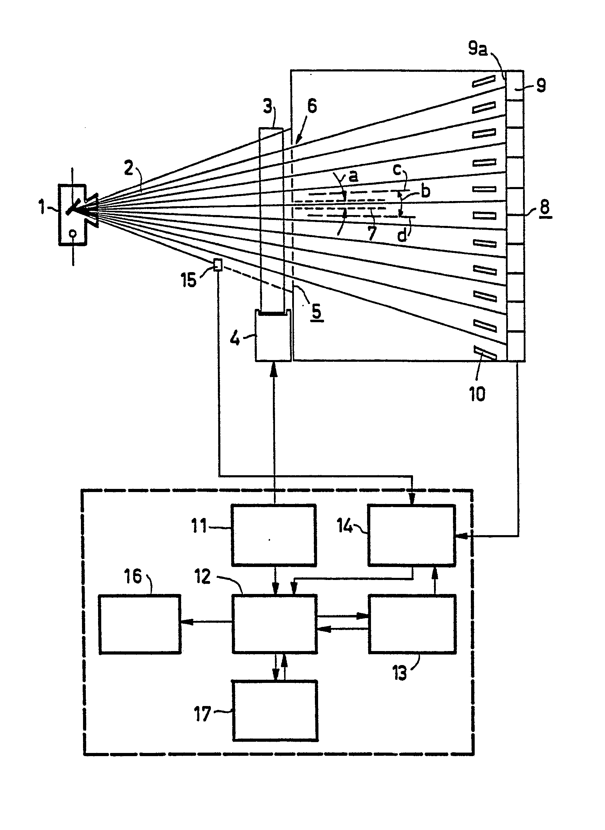

Die einzige Figur 1 stellt ein Ausführungsbeispiel der Untersuchungsanordnung nach der Erfindung dar. Als Strahlenquelle 1 ist eine Röntgenröhre vorgesehen, die ein räumlich divergentes (Primär-)Strahlenbündel 2 aussendet. Dieses Strahlenbündel 2 durchdringt einen zu untersuchenden Körper 3, z. B. ein auf Materialfehler zu untersuchendes Werkteil, das auf einer Verschiebevorrichtung 4 gelagert ist. Unmittelbar hinter dem Körper 3 befindet sich eine Blendenanordnung 5, die als Blendenplatte ausgebildet ist und eine Vielzahl von gleich großen Blendenöffnungen 6 aufweist.

Durch die Blendenöffnungen 6 treten sog. Abtaststrahlenbündel 7 hindurch, die von einer hinter der Blendenanordnung 5 angeordneten, zweidimensionalen Detektoranordnung 8 die aus einzelnen in einer zur Blendenplatte parallelen Ebene liegenden Strahlungsdetektoren 9 besteht, detektiert werden. Dabei ist jeweils einer Blendenöffnung 6 ein Strahlungsdetektor 9 zugeordnet. Sowohl die Blendenöffnungen 6 als auch die Strahlungsdetektoren 9 sind dabei in ihren jeweiligen Ebenen entlang von Zeilen und Spalten einer Matrix angeordnet. Die Größe der Blendenöffnungen 6 ist so gewählt, daß für jeweils eine Richtung die Öffnungswinkel a der Abtaststrahlenbündel 7 wesentlich kleiner sind als die halben Meßwinkel b der den Abtaststrahlenbündeln 7 zugeordneten Strahlungsdetektoren 9. Ein Meßwinkel bildet dabei den Winkel zwischen zwei vom Fokus der Strahlenquelle 1 ausgehenden Schenkeln c, d eines Dreiecks, dessen Basis die Abmessung eines Strahlungsdetektors 9 in Verschieberichtung ist.

Die Blendenanordnung 5 besteht beispielsweise aus einer auf einer strahlungsdurchlässigen Trägerplatte aufliegenden, mit Blendenöffnungen versehenen Bleifolie. Trägerplatte und Bleifolie sind dabei mit einer dünnen Aluminiumschicht versiegelt. In einer speziellen Ausführungsform sind die Blendenöffnungen 6 z. B. quadratisch ausgebildet und $0,1 \times 0,1$ m² groß, wobei der Abstand zwischen zwei Blendenöffnungen 6 ein Vielfaches ihrer Ausdehnung beträgt. Die Blendöffnungen 6 können aber auch anders, z. B. rechteckig oder kreisförmig, ausgebildet sein.

In einem Abstand von einem Meter zur Blendenanordnung 5 liegen die Detektoren 9, die entsprechend dem Gitter der Blendenöffnungen 6 angeordnet sind und jeweils eine quadratische, strahlungsempfindliche Eingangsfläche 9a von $1 \times 1$ cm² aufweisen. Die Detektoren 9 liegen ferner so, daß sie von den jeweiligen durch die Blendenöffnungen 6 hindurchtretenden Röntgenstrahlen 7 mittig getroffen werden. Zur Abschirmung von Streustrahlung, die im Körper und in der Blendenanordnung 5 entsteht, ist weiterhin ein Streustrahlenraster 10 vorgesehen, das dicht vor der Detektoranordnung 8 liegt.

Um nun von dem Körper 3 ein lückenloses Projektionsbild (Schattenbild) herstellen zu können, wird dieser im Strahlenbündel 2 parallel zur Blendenanordnung 5 verschoben, was mit Hilfe der Verschiebevorrichtung 4 durchgeführt wird. Auf diese Weise werden durch die jeweiligen Blendenöffnungen 6 unterschiedliche Bereiche des Körpers 3 mittels der Abtaststrahlenbündel 7 auf die entsprechenden Detektoren 9 nacheinander abgebildet und die Absorption entlang dieser Strahlenbündel gemessen. Die Bewegung des Körpers 3 im Strahlenbündel 2 hat den Vorteil, daß sich die Aufnahmegeometrie der Untersuchungsanordnung nicht verändert und z. B. zeitlich konstante Strahlinhomogenitäten, die bei der Erzeugung des Projektionsbildes mit Hilfe der Ausgangssignale der Strahlungsdetektoren 9 zu berücksichtigen sind, nur einmal ermittelt zu werden brauchen.

Die Verschiebung des Körpers 3 erfolgt dabei um solche Beträge, die der Ausdehnung der Blendenöffnungen 6 in Richtung der Verschiebung entsprechen. Bei matrixförmig angeordneten, quadratischen Blendenöffnungen 6 kann die Verschiebung z. B. in Zeilen und Spaltenrichtung um jeweils die Seitenlänge einer Blendenöffnung 6 erfolgen, wenn die Seiten parallel bzw. senkrecht zu den Zeilen oder Spalten verlaufen. Dabei kann die Verschiebung mäanderförmig ausgeführt werden.

Die Steuerung der Verschiebevorrichtung 4 wird mit Hilfe eines Steuermoduls 11 vorgenommen, das, entsprechend einer vorgewählten Verschiebung des Körpers, die Verschiebevorrichtung 4 in geeigneter Weise ansteuert. Die Steuersignale werden gleichzeitig an eine Recheneinheit 12 weitergeleitet, die die Koordinaten der nach jeder Verschiebung abzutastenden Körperbereiche bzw. Abtaststrahlenbündel errechnet und die Zuordnung der entsprechenden Meßdaten (Absorptionswerte) zu den Koordinaten übernimmt. Anschließend werden die Meßdaten und die Koordinaten in einem Speicher 13 gespeichert. Die Meßdaten können zuvor in einem Korrekturmodul 14 korrigiert werden. Diese Korrektur kann sowohl hinsichtlich zeitlich konstanter Strahlinhomogenitäten (z. B. Abfall der Strahlungsdichte an den Rändern des Strahlungskegels) als auch zur Kompensation zeitlicher Schwankungen der Strahlintensität erfolgen. Für den letzteren Fall ist ein Referenzdetektor 15 im Strahlenbündel 2 angeordnet, der seine Ausgangssignale an das Korrekturmodul 14 weiterleitet. Beim Prüfen kontrastloser Objekte auf kleine Fehler, die sich als Kontraste abzeichnen, kann auf den Referenzdetektor 15 verzichtet werden. Unter der Annahme, daß nur wenige Fehler in einem Bildausschnitt auftreten, stellt dann die Summe aller Meßwerte bei einer hinreichend großen Zahl von Detektoren eine im wesentlichen nur noch von der zeitlichen Schwankung der Strahldichte abhängige Größe dar, die zur Korrektur verwendet wird.

Zur Darstellung eines maßstabgetreuen Projektionsbildes des Körpers 3 werden die im Bildspeicher 13 gespeicherten Meßdaten mittels der Recheneinheit 12 einem Sichtgerät 16 (z. B. Monitor oder Hardcopy-Gerät) zugeleitet, in welchem sie unter Berücksichtigung der Lagekoordinaten der jeweiligen Abtaststrahlenbündel zusammengefügt werden. Die Meßdaten können aber auch mit entsprechenden Daten eines Musterkörpers (Vergleichskörpers), die in einem Musterspeicher 17 gespeichert sind, verglichen oder auf andere Art weiterverarbeitet werden. Die Unterschiede zwischen dem Musterkörper und dem tatsächlich untersuchten Körper 3 können dann z. B. auf dem Sichtgerät 16 dargestellt werden.

Zur Aufnahme des Projektionsbildes eines Körpers ist es natürlich auch möglich, die Strahlenquelle 1 sowie die Blenden- und Detektoranordnung 8 relativ zum ruhenden Körper 3 zu verschieben. Die Verschiebevorrichtung 4 ist dann z. B. mit einem gemeinsamen Träger (nicht dargestellt) der Elemente 1, 5 und 8 mechanisch zu koppeln. Ebenso kann nur die Blendenanordnung 5 relativ zum ruhenden Körper 3 und zu den ruhenden Elementen 1 und 8 verschoben werden, allerdings nur so weit, daß die von den Blendenanordnung 5 ausgeblendeten Strahlenbündel nicht auf die Ränder der Strahlungsdetektoren 9 auftreffen.

**Patentansprüche**

1. Vorrichtung zur nichtmedizinischen Untersuchung eines Körpers, mit einer Röntgenstrahlenquelle (1), die den Körper durchstrahlt, einer Detektoranordnung mit wenigstens einem Strahlungsdetektor (9) und mit einer Verschiebevorrichtung, die während der Messung eine Translationsbewegung wenigstens zwischen dem Körper und einer Blendenanordnung (5) erzeugt, durch die jedem Strahlungsdetektor eine Blendenöffnung (6) zugeordnet wird, die ein Abtaststrahlenbündel (7) mit einem Öffnungswinkel (a) hindurchläßt, der in Verschieberichtung kleiner ist als der durch den zugeordneten Strahlungsdetektor und die Röntgenstrahlen definierte Meßwinkel (b), dadurch gekennzeichnet, daß die Detektoranordnung (8) aus einem zweidimensionalen Feld von in Zeilen und Spalten einer Matrix angeordneten Strahlungsdetektoren (9) besteht und die Verschiebevorrichtung (4) Translationsbewegungen in zwei zueinander senkrechten Richtungen gestattet.

2. Vorrichtung nach Anspruch 1 mit einer Röntgenstrahlenquelle, einer Detektoranordnung, einer Verschiebevorrichtung und einer Blendenanordnung, dadurch gekennzeichnet, daß die Blendenanordnung (5) als Blendenplatte ausgebildet und zwischen der Detektoranordnung (8) und dem Körper (3) in dessen unmittelbarer Nähe angeordnet ist.

3. Vorrichtung nach Anspruch 1 mit einer Röntgenstrahlenquelle, einer Detektoranordnung, einer Verschiebevorrichtung und einer Blendenanordnung, dadurch gekennzeichnet, daß die Blendenöffnungen (6) rechteckig, quadratisch oder kreisförmig sind.

4. Vorrichtung nach Anspruch 1 mit einer Röntgenstrahlenquelle, einer Detektoranordnung, einer Verschiebevorrichtung und einer Blendenanordnung, dadurch gekennzeichnet, daß die Strahlenquelle (1), die Blendenanordnung (5) und die Detektoranordnung (8) fest zueinander positioniert sind, und daß zwischen ihnen und dem Körper (3) mittels der Verschiebevorrichtung eine Relativverschiebung erzeugbar ist.

5. Vorrichtung nach Anspruch 1 mit einer Röntgenstrahlenquelle, einer Detektoranordnung, einer Verschiebevorrichtung und einer Blendenanordnung, dadurch gekennzeichnet, daß nur die Blendenanordnung (5) verschiebbar ist.

6. Vorrichtung nach Anspruch 1 mit einer Röntgenstrahlenquelle, einer Detektoranordnung, einer Verschiebevorrichtung und einer Blendenanordnung, dadurch gekennzeichnet, daß im Strahlengang zwischen der Strahlenquelle (1) und dem Objekt (3) zusätzlich ein Referenz-Strahlungsdetektor (15) angeordnet ist.

7. Vorrichtung nach Anspruch 1 mit einer Röntgenstrahlenquelle, einer Detektoranordnung, einer Verschiebevorrichtung und einer Blendenanordnung, dadurch gekennzeichnet, daß im Strahlengang zwischen der Blendenanordnung (5) und der Detektoranordnung (8) zusätzlich ein Streustrahlenraster (10) angeordnet ist.

## Claims

1. An arrangement for non-medical examination of a body, comprising an X-ray source (1) which irradiates the body, a detector device with at least one radiation detector (9) and a displacement device which produces a translatory movement at least between the body and a diaphragm device (5) during the measurement, said diaphragm device assigning to each radiation detector an aperture (6) which transmits a scanning radiation beam (7) which has an angle of aperture (a) which is smaller, measured in the displacement direction, than the measurement angle (b) defined by the associated radiation detector and the X-rays, characterized in that the detector device (8) consists of a twodimensional field of radiation detectors (9) which are arranged in rows and columns of a matrix, the displacement device (4) enabling translatory movements in two mutually perpendicular directions.

2. An arrangement as claimed in Claim 1 which comprises an X-ray source, a detector device, a displacement device and a diaphragm device, characterized in that the diaphragm device (5) is constructed as a diaphragm plate which is arranged between the detector device (8) and the body (3) in the direct vicinity of the body.

3. An arrangement as claimed in Claim 1 which comprises an X-ray source, a detector device, a displacement device and a diaphragm device, characterized in that the apertures (6) have a rectangular, square or circular shape.

4. An arrangement as claimed in Claim 1, which comprises an X-ray source, a detector device, a displacement device and a diaphragm device, characterized in that the radiation source (1), the diaphragm device (5) and the detector device (8) are positioned to be stationary with respect to one another, the displacement device enabling a relative displacement between these devices and the body (3).

5. An arrangement as claimed in Claim 1 which comprises an X-ray source, a detector device, a displacement device and a diaphragm device, characterized in that only the diaphragm device (5) is displaceable.

6. An arrangement as claimed in Claim 1 which comprises an X-ray source, a detector device, a displacement device and a diaphragm device, characterized in that in the beam path between the radiation source (1) and the object (3) there is additionally arranged a reference radiation detector (15).

7. An arrangement as claimed in Claim 1 which comprises an X-ray source, a detector device, a displacement device and a diaphragm device, characterized in that in the beam path between the diaphragm device (5) and the detector device (8) there is additionally arranged a scatter radiation grid (10).

## Revendications

1. Dispositif pour l'examen non médical d'un corps comportant une source de rayons X (1) qui traversent le corps, un dispositif de détection comportant au moins un détecteur de rayonnement (9) et un dispositif de déplacement qui, pendant la mesure, produit un mouvement de translation au moins entre le corps et un dispositif à diaphragmes (5) grâce auquel à chaque détecteur de rayonnement est associé un diaphragme (6) qui laisse passer un faisceau de rayons d'exploration (7) avec un angle d'ouverture (a) qui est inférieur, dans le sens du déplacement, à l'angle de mesure (b) défini par le détecteur de rayonnement associé et les rayons X, caractérisé en ce que le dispositif de détection (8) est formé d'un champ bidimensionnel de détecteurs de rayonnement (9) disposés suivant les lignes et les colonnes d'une matrice et le dispositif de déplacement (4) permet des mouvements de translation dans deux directions perpendiculaires l'une à l'autre.

2. Dispositif suivant la revendication 1 comportant une source de rayons X, un dispositif de détection, un dispositif de déplacement et un dispositif à diaphragmes, caractérisé en ce que le dispositif à diaphragmes (5) a la forme d'une plaque à diaphragmes et est disposé entre le dispositif de détection (8) et le corps (3) au voisinage immédiat de celui-ci.

3. Dispositif suivant la revendication 1 comportant une source de rayons X, un dispositif de détection, un dispositif de déplacement et un dispositif à diaphragmes, caractérisé en ce que les ouvertures des diaphragmes (6) sont rectangulaires, carrées ou circulaires.

4. Dispositif suivant la revendication 1 comportant une source de rayons X, un dispositif de détection, un dispositif de déplacement et un dispositif formant diaphragme, caractérisé en ce que la source de rayons (1), le dispositif à diaphragmes (5) et le dispositif de détection (8) sont positionnés de manière fixe l'un par rapport à l'autre et entre ces éléments et le corps (3), un déplacement relatif peut être produit au moyen du dispositif de déplacement.

5. Dispositif suivant la revendication 1 comportant une source de rayons X, un dispositif de détection, un dispositif de déplacement et un dispositif à diaphragmes, caractérisé en ce que seul le dispositif à diaphragmes (5) peut être déplacé.

6. Dispositif suivant la revendication 1 comportant une source de rayons X, un dispositif de détection, un dispositif de déplacement et un dispositif à diaphragmes, caractérisé en ce que dans le trajet des rayons entre la source de rayons (1) et l'objet (3) est prévu, en outre, un détecteur de rayonnement de référence (15).

7. Dispositif suivant la revendication 1 comportant une source de rayons X, un dispositif de détection, un dispositif de déplacement et un dispositif à diaphragmes, caractérisé en ce que dans le trajet des rayons entre le dispositif à diaphragmes (5) et le dispositif de détection (8) est disposée, en outre, une grille antidiffusante (10).